# FASCICULE DE BREVET EUROPEEN

(11) **EP 3 129 076 B1**
(45) Date de publication et mention de la délivrance du brevet: **26.05.2021**
(21) Numéro de dépôt: 15725726.2
(22) Date de dépôt: 13.04.2015
(51) Int. Cl.: A61L 29/06, C08L 77/00

(54) **UTILISATION DE PEBA À BLOCS LONGS POUR LA FABRICATION DE TOUT OU PARTIE D'UN CATHÉTER**
VERWENDUNG VON PEBA MIT LANGEN BLÖCKEN ZUR HERSTELLUNG EINES KOMPLETTEN KATHETERS ODER TEILS DAVON
USE OF PEBA HAVING LONG BLOCKS FOR THE MANUFACTURE OF ALL OR PART OF A CATHETER

(30) Priorité: 11.04.2014 FR 1453224
(43) Date de publication de la demande: 15.02.2017
(73) Titulaire: Arkema France, 92700 Colombes (FR)
(72) Inventeur: EUSTACHE, René-Paul, 27170 Combon (FR); MALET, Frédéric, 69007 Lyon (FR); GAMACHE, Eric, 76000 Rouen (FR); REYNA-VALENCIA, Alejandra, 27930 Aviron (FR); RAULINE, Damien, 27270 Saint-Quentin-des-Isles (FR)
(86) Numéro de dépôt international: PCT/FR2015/050978
(87) Numéro de publication internationale: WO 2015/155489

(56) Documents cités:
- EP-A2- 0 156 035
- WO-A1-2009/062711
- WO-A2-99/13924
- US-A1- 2011 288 478

## Description

### DOMAINE DE L'INVENTION

La présente invention concerne plus particulièrement un élément gonflable de cathéter intravasculaire, tel qu'un ballon ou ballonnet.

L'invention a plus particulièrement pour objet l'utilisation de nouvelles compositions de polymères thermoplastiques pour la fabrication de ballons de cathéters médicaux, qui combinent d'excellentes propriétés de « compliance » et de résistance à l'éclatement, telles que définies ci-après. Néanmoins, on pourra envisager de réaliser une autre partie, voire la totalité, du cathéter avec la composition selon l'invention.

### ARRIERE-PLAN TECHNIQUE

Dans la suite, l'invention est présentée en relation avec les ballons d'un cathéter, leurs problématiques spécifiques et leurs fonctionnalités, mais il est bien entendu que la composition selon l'invention est à même de répondre aux exigences techniques d'autres parties d'un cathéter, notamment la tige.

Les cathéters de dilatation à ballonnet sont utilisés en angioplastie coronarienne transluminale percutanée (ACTP ou PTCA), procédé largement utilisé pour le traitement de la maladie coronarienne. Dans la procédure ACTP, un cathéter de dilatation à ballonnet est avancé dans l'artère coronaire d'un patient et le ballonnet du cathéter est gonflé à l'intérieur de la région sténosée de l'artère du patient pour ouvrir le passage artériel et augmenter ainsi le débit sanguin. Généralement, la forme et le diamètre du ballonnet gonflé sont prédéterminés et correspondent approximativement au diamètre d'origine du lumen de l'artère dilatée normalement, de façon à dilater l'artère mais sans élargir plus sa paroi. Une fois le ballonnet dégonflé, alors le débit sanguin dans l'artère ainsi dilatée reprend et le cathéter de dilatation peut être retiré de celle-ci.

Pour prévenir le taux de resténose et renforcer l'espace ainsi dilaté, les médecins implantent souvent une prothèse intravasculaire, généralement appelée stent, à l'intérieur de l'artère sur le site de la lésion. Les stents peuvent également être utilisés pour réparer les vaisseaux ou renforcer une section affaiblie d'un vaisseau. Les stents sont généralement placés à l'endroit souhaité à l'intérieur d'une artère coronaire contractée ou rétrécie, au moyen d'un ballon de cathéter semblable à un ballon de cathéter d'angioplastie, et élargis en un plus grand diamètre par l'expansion du ballon. Le ballon est ensuite dégonflé pour retirer le cathéter et le stent est mis en place dans l'artère au site de la lésion ainsi dilaté.

Les ballons pour cathéters de dilatation de l'art antérieur, généralement utilisés dans les procédures d'angioplastie, sont formés de matériaux polymères non élastiques tels que le chlorure de polyvinyle (PVC), le polyéthylène (PE), le polyéthylène téréphtalate (PET), les ionomères polyoléfiniques, et le polyamide (PA). Un avantage de ces matériaux inélastiques, lorsqu'ils sont utilisés dans des ballons du cathéter est que la résistance à la traction, et par conséquent, la pression moyenne à la rupture, du ballon est élevée.

Les ballons de cathéters doivent en effet avoir une résistance élevée à la traction afin d'exercer une pression suffisante sur le vaisseau sténosé pour ouvrir efficacement la circulation du patient. De plus, un ballon de haute résistance à la traction peut être gonflé à des pressions élevées sans risque que le ballon éclate pendant la pressurisation. Enfin, l'épaisseur de paroi d'un ballon de haute résistance peut être amincie, afin de diminuer le profil du cathéter sans risque d'éclatement. Il existe en effet un rapport direct entre la pression d'éclatement et la contrainte en traction (voir sur le sujet l'article « Médical Device and Diagnostic Industry: New extrusion techniques advance catheter design », by Byron Flagg (Putnam Plastics), http://www.mddionline.com/article/new-extrusion-techniques-advance-catheter-design).

L'inconvénient de ces matériaux inélastiques ayant le moins d'élasticité est leur manque de « compliance ». Ces matériaux sont en effet classés en matériaux «non-conformes» (ou « non-compliant ») et matériaux «semi-conformes» (semi-compliant), et incluent notamment le PET et le polyamide. Le matériau non conforme présente peu d'expansion en réponse à des niveaux croissants de pression de gonflage. Pour ces matériaux non conformes, à cause de la capacité limitée du ballon à augmenter son diamètre, le ballon gonflé doit être suffisamment grand pour que, une fois gonflé, le ballon ait un diamètre de travail suffisant pour compresser la sténose et ouvrir la circulation du patient. Cependant, un grand profil de ballon non conforme peut rendre le cathéter difficile à avancer dans le système vasculaire étroit du patient car, dans un état non gonflé, ces ballons forment des ailes de forme plate (comme une crêpe) qui s'étendent radialement vers l'extérieur. Par conséquent, la présente invention a donc pour but de fournir un matériau pour ballon de cathéter présentant une meilleure « conformité » ou « compliance ». Des ballons formés de matériaux « conformes » ou « compliant » ont une souplesse accrue, ce qui améliore la capacité de la sonde à suivre le système vasculaire tortueux du patient et à traverser la sténose, et permet de positionner correctement le ballonnet à l'endroit de la sténose. La souplesse d'un ballon est exprimée par le module d'élasticité en flexion du ballonnet. Un ballon relativement souple (ou mou) présente un module d'élasticité en flexion relativement faible, c'est-à-dire inférieur à environ 1000 MPa.

D'autres matériaux polymères, en particulier les copolymères à blocs polyamide et blocs polyéther (PEBA), sont utilisés dans la fabrication de cathéters pour améliorer leur aspect glissant et permettre ainsi une insertion dans les vaisseaux plus confortable pour le patient. Les PEBA peuvent également être utilisés pour la fabrication de ballons de cathéter, et ces matériaux qui présentent des propriétés intéressantes de haute résistance à la traction, haute élongation et de faible module de flexion, permettent de répondre en partie aux exigences précitées WO99/13924 décrit un copolymère utilisé pour la fabrication d'un élément gonflable de catheter. Ledit copolymère comprend des blocs polyéther et des blocs polyamide ayant les caractéristiques suivantes: la masse moléculaire moyenne en nombre des blocs PE est comprise entre 650 et 1000 g/mol et la masse moléculaire moyenne en nombre des blocs PA est comprise entre 300 et 15000 g/mol.

La compliance ou conformité des matériaux PEBA actuellement utilisés se matérialise par une courbe Contrainte (MPa) - Déformation (%), dont le profil est caractérisé par un premier segment, généralement linéaire « compliant » et un deuxième segment « non-compliant » (ne suivant pas une déformation linéaire) séparés par un segment de transition correspondant au seuil de la courbe contrainte déformation.

Il s'avère que les ballons en PEBA présentent parfois une épaisseur de paroi non uniforme, ce qui n'est pas acceptable pour les applications de cathéter à cause des risques d'éclatement au cours de leur gonflage.

La présente invention a donc pour but de fournir un procédé pour améliorer et faciliter la fabrication de ballons ou ballonnets à base de PEBA, pour qu'ils présentent une paroi d'épaisseur la plus uniforme possible. Cette propriété est souhaitable pour limiter les risques d'éclatement au cours de leur gonflage, que ce soit pendant leur fabrication ou pendant leur utilisation.

La présente invention vise en particulier à fournir des matériaux « compliant », permettant un meilleur contrôle de l'uniformité de l'épaisseur de paroi du ballon de cathéter, et donc de réduire la quantité de ballons mis au rebus. La présente invention a également pour but de fabriquer des ballons ou ballonnets aux parois les plus fines possibles, permettant à la fois d'utiliser des cathéters à ballons les moins invasifs possibles lors de leur insertion dans les vaisseaux, d'améliorer la sécurité d'utilisation de ces ballons en limitant leur risque d'éclatement, tout en utilisant moins de matière première polymère.

La Demanderesse a maintenant trouvé qu'un choix spécial de PEBA, présentant un profil de courbe particulier, permet de contrôler facilement l'uniformité de l'épaisseur de paroi du ballon au cours de sa fabrication. De manière surprenante, certains matériaux PEBA qui présentent une courbe de compliance dont le segment intermédiaire est le plus court possible voir inexistant entre le segment compliant et le segment non-compliant, permettent de fabriquer facilement des ballons d'épaisseur de paroi uniforme. Par « épaisseur de paroi uniforme », on entend une paroi qui a la même épaisseur sur toute sa surface.

### DESCRIPTION DE L'INVENTION

Ceci est accompli grâce l'utilisation selon l'invention de copolymères à blocs polyéther et blocs polyamide dits « à blocs longs » selon l'invention, tels que définis ci-après d'après la masse moléculaire moyenne en nombre des blocs PA et PE.

La présente invention a donc pour objet l'utilisation d'un copolymère à blocs polyéther et blocs polyamide pour la fabrication d'un élément gonflable de cathéter, tel qu'un ballon de cathéter, à résistance à l'éclatement améliorée, dans laquelle ledit copolymère a les caractéristiques suivantes :
- masse moléculaire moyenne en nombre des blocs PE supérieure à 500 g/mol,
- masse moléculaire moyenne en nombre des blocs PA supérieure à 10000 g/mol; le copolymère comprend des blocs polyéther composés majoritairement à base de PTMG.

Ce résultat est particulièrement surprenant dans la mesure où les polymères thermoplastiques élastomères à blocs longs sont généralement beaucoup plus difficiles à synthétiser suite à l'incompatibilité plus grande des blocs en fonction de leur longueur - plus la longueur des chaînes polymères, plus précisément en l'espèce PE et PA, sont longues, plus elles seront incompatibles - et difficile à mettre en œuvre sans présenter d'avantages notables par rapport aux élastomères classiques à blocs courts.

Contrairement à l'enseignement général de l'homme du métier, les inventeurs ont remarqué que la présence de ces blocs PA et PE particulièrement longs permet d'améliorer la tenue à l'éclatement, ainsi que la rigidité, pour des masses moléculaires équivalentes sans impact concernant la mise en œuvre.

La présente invention permet de surmonter les inconvénients de l'état de la technique en utilisant un type particulier de polymères thermoplastiques élastomères, pour la fabrication de matériaux combinant les 3 qualités requises pour une utilisation dans les ballons de cathéter: souplesse, résistance à l'éclatement, et « compliance », telles que définies ci-dessous :
- souplesse, c'est-à-dire de module d'élasticité en flexion inférieur à 1000 MPa, et supérieur à 525 MPa, mesuré selon la norme internationale ISO 178.
- résistance à l'éclatement, en particulier résistant à la traction, c'est-à-dire de contrainte au seuil supérieure à 20 MPa et de déformation au seuil supérieure à 22%, mesurée selon la norme ISO 527, et
- « compliance », c'est-à-dire dont l'écart entre la contrainte au seuil et la contrainte au plateau d'écoulement est inférieur à 0,5 MPa, et dont le rapport entre la déformation au seuil et la déformation au plateau d'écoulement est supérieur à 0,5, les contraintes et déformations étant mesurées selon la norme ISO 527.

S'agissant des copolyéther bloc amides, encore appelés copolymères à blocs polyéther et blocs polyamide, soit en abrégé « PEBA », ils résultent de la polycondensation de blocs polyamides à extrémités réactives avec des blocs polyéthers à extrémités réactives, telles que, entre autres :
1) blocs polyamides à bouts de chaîne diamines avec des blocs polyoxyalkylènes à bouts de chaînes dicarboxyliques ;
2) blocs polyamides à bouts de chaînes dicarboxyliques avec des blocs polyoxyalkylènes à bouts de chaînes diamines, obtenues par cyanoéthylation et hydrogénation de blocs polyoxyalkylène alpha-oméga dihydroxylées aliphatiques appelées polyétherdiols ;
3) blocs polyamides à bouts de chaînes dicarboxyliques avec des polyétherdiols, les produits obtenus étant, dans ce cas particulier, des polyétheresteramides.

Les blocs polyamides à bouts de chaînes dicarboxyliques proviennent, par exemple, de la condensation de précurseurs de polyamides en présence d'un diacide carboxylique limiteur de chaîne. Les blocs polyamides à bouts de chaînes diamines proviennent par exemple de la condensation de précurseurs de polyamides en présence d'une diamine limiteur de chaîne.

Les polymères à blocs polyamides et blocs polyéthers peuvent aussi comprendre des motifs répartis de façon aléatoire.

On peut utiliser avantageusement trois types de blocs polyamides.

Selon un premier type, les blocs polyamides proviennent de la condensation d'un diacide carboxylique, en particulier ceux ayant de 4 à 20 atomes de carbone, de préférence ceux ayant de 6 à 18 atomes de carbone et d'une diamine aliphatique ou aromatique, en particulier celles ayant de 2 à 20 atomes de carbone, de préférence celles ayant de 6 à 14 atomes de carbone.

A titre d'exemples d'acides dicarboxyliques, on peut citer l'acide 1,4-cyclohexyldicarboxylique, les acides butanedioïque, adipique, azélaïque, subérique, sébacique, dodécanedicarboxylique, octadécanedicarboxylique et les acides téréphtalique et isophtalique, mais aussi les acides gras dimérisés.

A titre d'exemples de diamines, on peut citer la tétraméthylène diamine, l'hexaméthylènediamine, la 1,10-décaméthylènediamine, la dodécaméthylènediamine, la triméthylhexaméthylène diamine, les isomères des bis-(4-aminocyclohexyl)-méthane (BACM), bis-(3-méthyl-4-aminocyclohexyl)méthane (BMACM), et 2-2-bis-(3-méthyl-4-aminocyclohexyl)-propane (BMACP), et para-amino-di-cyclo-hexyl-méthane (PACM), et l'isophoronediamine (IPDA), la 2,6-bis-(aminométhyl)-norbornane (BAMN) et la pipérazine (Pip).

Avantageusement, on a des blocs PA4.12, PA4.14, PA4.18, PA6.10, PA6.12, PA6.14, PA6.18, PA9.12, PA10.10, PA10.12, PA10.14 et PA10.18.

Selon un deuxième type, les blocs polyamides résultent de la condensation d'un ou plusieurs acides alpha oméga aminocarboxyliques et/ou d'un ou plusieurs lactames ayant de 6 à 12 atomes de carbone en présence d'un diacide carboxylique ayant de 4 à 12 atomes de carbone ou d'une diamine. A titre d'exemples de lactames, on peut citer le caprolactame, l'oenantholactame et le lauryllactame. A titre d'exemples d'acide alpha omega amino carboxylique, on peut citer les acides aminocaproïque, amino-7-heptanoïque, amino-11- undécanoïque et amino-12-dodécanoïque.

Avantageusement les blocs polyamides du deuxième type sont en polyamide 11, en polyamide 12 ou en polyamide 6.

Selon un troisième type, les blocs polyamides résultent de la condensation d'au moins un acide alpha oméga aminocarboxylique (ou un lactame), au moins une diamine et au moins un diacide carboxylique.

Dans ce cas, on prépare les blocs polyamide PA par polycondensation :
- de la ou des diamines aliphatiques linéaires ou aromatiques ayant X atomes de carbone ;
- du ou des diacides carboxyliques ayant Y atomes de carbone ; et
- du ou des comonomères {Z}, choisis parmi les lactames et les acides alpha-oméga aminocarboxyliques ayant Z atomes de carbone et les mélanges équimolaires d'au moins une diamine ayant X1 atomes de carbone et d'au moins un diacide carboxylique ayant Y1 atomes de carbones, (X1, Y1) étant différent de (X, Y) ;
- ledit ou lesdits comonomères {Z} étant introduits dans une proportion pondérale allant jusqu'à 50%, de préférence jusqu'à 20%, encore plus avantageusement jusqu'à 10% par rapport à l'ensemble des monomères précurseurs de polyamide ;
- en présence d'un limiteur de chaîne choisi parmi les diacides carboxyliques.

Avantageusement, on utilise comme limiteur de chaîne le diacide carboxylique ayant Y atomes de carbone, que l'on introduit en excès par rapport à la stœchiométrie de la ou des diamines.

Selon une variante de ce troisième type les blocs polyamides résultent de la condensation d'au moins deux acides alpha,oméga aminocarboxyliques ou d'au moins deux lactames ayant de 6 à 12 atomes de carbone ou d'un lactame et d'un acide aminocarboxylique n'ayant pas le même nombre d'atomes de carbone en présence éventuelle d'un limiteur de chaîne. A titre d'exemple d'acide alpha omega amino carboxylique aliphatique, on peut citer les acides aminocaproïques, amino-7-heptanoïque, amino-11-undécanoïque et amino-12-dodécanoïque. A titre d'exemple de lactame, on peut citer le caprolactame, l'oenantholactame et le lauryllactame. A titre d'exemple de diamines aliphatiques, on peut citer l'hexaméthylènediamine, la dodécaméthylènediamine et la triméthylhexaméthylène diamine. A titre d'exemple de diacides cycloaliphatiques, on peut citer l'acide 1,4-cyclohexyldicarboxylique. A titre d'exemple de diacides aliphatiques, on peut citer les acides butane-dioïque, adipique, azélaïque, subérique, sébacique, dodécanedicarboxylique, les acides gras dimérisés (ces acides gras dimérisés ont de préférence une teneur en dimère d'au moins 98%; de préférence ils sont hydrogénés; ils sont commercialisés sous la marque Pripol® par la société Unichema, ou sous la marque Empol® par la société Henkel) et les polyoxyalkylènes - α,ω diacides. A titre d'exemple de diacides aromatiques, on peut citer les acides téréphtalique (T) et isophtalique (I). A titre d'exemple de diamines cycloaliphatiques, on peut citer les isomères des bis-(4-aminocyclohexyl)-méthane (BACM), bis-(3-méthyl-4-aminocyclohexyl)méthane (BMACM), et 2-2-bis-(3-méthyl-4-aminocyclohexyl)-propane(BMACP), et para-amino-di-cyclo-hexyl-méthane (PACM). Les autres diamines couramment utilisées peuvent être l'isophoronediamine (IPDA), la 2,6-bis-(aminométhyl)-norbornane (BAMN) et la pipérazine.

A titre d'exemples de blocs polyamides du troisième type, on peut citer les suivantes :
- 6.6/6 dans laquelle 6.6 désigne des motifs hexaméthylènediamine condensée avec l'acide adipique. 6 désigne des motifs résultant de la condensation du caprolactame.
- 6.6/6.10/11/12 dans laquelle 6.6 désigne l'hexaméthylènediamine condensée avec l'acide adipique. 6.10 désigne l'hexaméthylènediamine condensée avec l'acide sébacique. 11 désigne des motifs résultant de la condensation de l'acide aminoundécanoïque. 12 désigne des motifs résultant de la condensation du lauryllactame.

Les segments de polyamide peuvent également comprendre des polyamides aromatiques, mais dans ce cas des caractéristiques de compliance significativement plus faibles sont à prévoir.

Les blocs polyéthers sont constitués de motifs oxyde d'alkylène. Ces motifs peuvent être par exemple des motifs oxyde d'éthylène, des motifs oxyde de propylène ou tétrahydrofurane (qui conduit aux enchaînements polytétraméthylène glycol). On utilise ainsi des blocs PEG (polyethylène glycol) c'est à dire ceux constitués de motifs oxyde d'éthylène, des blocs PPG (propylène glycol) c'est à dire ceux constitués de motifs oxyde de propylène, des blocs PO3G (polytriméthylène glycol) c'est-à-dire ceux constitués de motifs polytriméthylène ether de glycol (de tels copolymères avec des blocs polytriméthylene ether sont décrits dans le document US6590065), et des blocs PTMG c'est à dire ceux constitués de motifs tetraméthylène glycols appelés aussi polytétrahydrofurane. Les copolymères PEBA peuvent comprendre dans leur chaîne plusieurs types de polyéthers, les copolyéthers pouvant être à blocs ou statistiques. On peut également utiliser des blocs obtenus par oxyéthylation de bisphenols, tels que par exemple le bisphenol A. Ces derniers produits sont décrits dans le brevet EP613919.

Les blocs polyéthers peuvent aussi être constitués d'amines primaires éthoxylées. A titre d'exemple d'amines primaires éthoxylées on peut citer les produits de formule : 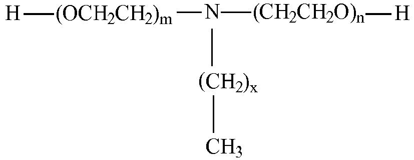 dans laquelle m et n sont compris entre 1 et 20 et x entre 8 et 18. Ces produits sont disponibles dans le commerce sous la marque Noramox® de la société CECA et sous la marque Genamin® de la société Clariant.

Les blocs souples polyéthers peuvent comprendre des blocs polyoxyalkylène à bouts de chaînes NH2, de telles blocs pouvant être obtenues par cyanoacétylation de blocs polyoxyalkylène alpha-oméga dihydroxylés aliphatiques appelées polyétherdiols. Plus particulièrement, on pourra utiliser les Jeffamines (Par exemple Jeffamine® D400, D2000, ED 2003, XTJ 542, produits commerciaux de la société Huntsman, également décrites dans les documents de brevets JP2004346274, JP2004352794 et EP1482011).

Les blocs polyétherdiols sont soit utilisés tels quels et copolycondensés avec des blocs polyamides à extrémités carboxyliques, soit ils sont aminés pour être transformés en polyéther diamines et condensés avec des blocs polyamides à extrémités carboxyliques. La méthode générale de préparation en deux étapes des copolymères PEBA ayant des liaisons ester entre les blocs PA et les blocs PE est connue et est décrite, par exemple, dans le brevet français FR2846332. La méthode générale de préparation des copolymères PEBA de l'invention ayant des liaisons amide entre les blocs PA et les blocs PE est connue et décrite, par exemple dans le brevet européen EP1482011. Les blocs polyéther peuvent être aussi mélangés avec des précurseurs de polyamide et un limiteur de chaîne diacide pour faire les polymères à blocs polyamides et blocs polyéthers ayant des motifs répartis de façon statistique (procédé en une étape).

Bien entendu, la désignation PEBA dans la présente description de l'invention se rapporte aussi bien aux Pebax® commercialisés par Arkema, aux Vestamid® commercialisés par Evonik®, aux Grilamid® commercialisés par EMS, qu'aux Kellaflex® commercialisés par DSM ou à tout autre PEBA d'autres fournisseurs.

Avantageusement, les copolymères PEBA ont des blocs PA en PA 6, en PA 11, en PA 12, PA 6.12, en PA 6.6/6, en PA 10.10 et/ou en PA 6.14, de préférence des blocs PA 11 et/ou PA 12 ; et des blocs PE en PTMG, en PPG et/ou en PO3G. Les PEBA à base de blocs PE constitués majoritairement de PTMG sont à ranger dans la gamme des PEBA hydrophobes.

Avantageusement, ledit PEBA utilisé dans la composition selon l'invention est obtenu au moins partiellement à partir de matières premières bio-ressourcées.

Par matières premières d'origine renouvelable ou matières premières bio-ressourcées, on entend des matériaux qui comprennent du carbone bio-ressourcé ou carbone d'origine renouvelable. En effet, à la différence des matériaux issus de matières fossiles, les matériaux composés de matières premières renouvelables contiennent du ¹⁴C. La « teneur en carbone d'origine renouvelable » ou « teneur en carbone bio-ressourcé » est déterminée en application des normes ASTM D 6866 (ASTM D 6866-06) et ASTM D 7026 (ASTM D 7026-04). A titre d'exemple, les PEBA à base de polyamide 11 proviennent au moins en partie de matières premières bio-ressourcées et présentent une teneur en carbone bio-ressourcé d'au moins 1%, ce qui correspond à un ratio isotopique de ¹²C/¹⁴C d'au moins 1,2 x 10¹⁴. De préférence, les PEBA selon l'invention comprennent au moins 50% en masse de carbone bio-ressourcé sur la masse totale de carbone, ce qui correspond à un ratio isotopique ¹²C/¹⁴C d'au moins 0,6.10⁻¹². Cette teneur est avantageusement plus élevée, notamment jusqu'à 100%, qui correspond à un ratio isotopique ¹²C/¹⁴C de 1,2 x 10⁻¹², dans le cas de PEBA à blocs PA 11 et blocs PE comprenant du PTMG.

Avantageusement, lesdits blocs PE représentent 1-20% en poids, de préférence de 1-10% en poids, sur le poids total du copolymère, et lesdits blocs PA représentent 80-99% en poids, de préférence de 90-99% en poids, sur le poids total du copolymère.

Avantageusement, la dureté shore D (selon la norme internationale ISO 868) du copolymère est supérieure ou égale à 72, de préférence comprise dans la gamme de 72 à 76.

Le rapport pondéral du polyamide au polyéther dans le PEBA utilisé dans l'invention est entre 9 et 50, de préférence supérieur à 15.

Avantageusement, la masse moléculaire moyenne en nombre des blocs PE est supérieure à 600 g/mol, de préférence comprise dans la gamme de 600 à 2000 g/mol, de préférence de 650 à 1000 g/mol.

Avantageusement, la masse moléculaire moyenne en nombre des blocs PA est comprise dans la gamme de 12000 à 32000 g/mol, de préférence de 13000 à 25000 g/mol. Ce domaine de masse moléculaire est choisi de manière à satisfaire le maintien des niveaux de souplesse souhaitée pour la composition selon l'invention. Les viscosités inhérentes de ces polymères selon l'invention sont supérieures à 1,5.

De manière avantageuse, le module d'élasticité en flexion du copolymère (mesuré selon la norme ISO 178) est supérieur à 525 MPa. Le module d'élasticité en flexion du copolymère est de préférence compris dans la gamme de 600 à 900 MPa. Ces domaines de valeurs pour le module d'élasticité en flexion du copolymère sont choisis de telle manière qu'elles sont particulièrement adaptées pour d'une part autoriser l'avancée du cathéter dans les artères et d'autre part autoriser le déploiement du stent associé au cathéter lors du gonflage du ballon pour le positionnement dudit stent à son emplacement dédié.

Avantageusement, les blocs PA sont formés à partir d'au moins un monomère choisi parmi : 6, 11, 12, 4.6, 4.12, 4.14, 4.18, 6.6, 6.10, 6.12, 6.14, 6.18, Pip.10, 9.6, 9.12, 10.10, 10.12, 10.14, 10.18, 10.36, 10.T, 6.T, 9.T, MXD.6, MXD.10, B.10, B.12, B.14, B.18, B.36, P.10, P.12, P.14, P.18, P.36, leurs copolymères statistiques et/ou à blocs, et leurs mélanges.

Le copolymère comprend des blocs polyéther composés majoritairement à base de PTMG, et optionnellement au moins un autre polyalkylène éther polyol, tel que le PEG, le PPG, le PO3G, les polyéthers contenant des séquences polyoxyalkylène à bouts de chaîne NH2, leurs copolymères statistiques et/ou à blocs, et leurs mélanges, de préférence composés uniquement de PTMG.

La présente invention a également pour objet une composition polymérique thermoplastique à base de polyamide, ladite composition comprenant :
- de 30 à 100% en poids de copolymère conforme à l'invention,
- de 0 à 70% en poids d'au moins un autre polymère choisi parmi les polyamides, les PEBA autres que ceux utilisés selon l'invention, TPU, COPE, PVC, ABS, PS, PET, PETE, PVDF, ETFE, Polyimide, PEEK, PEKK, silicone, « silicon rubber » (gomme de silicone).
- de 0 à 40% d'additif, sur le poids total de la composition.

Dans le cas classique, il faut noter que les additifs ne sont pas présents à plus de 20% dans la composition selon l'invention. Néanmoins, dans l'hypothèse en particulier où des additifs fonctionnels (traceurs) du type Ba₂S0₄ (sulfate de baryum) et/ou du WC (carbure de tungstène) sont utilisés, la quantité d'additifs peut être supérieure à 20% en poids de la composition.

De préférence, l'additif est choisi parmi des agents colorants, notamment les pigments, les colorants, les pigments à effet, tels que les pigments diffractant, les pigments interférentiels, tels que les nacres, les pigments réfléchissants et leurs mélanges; des agents anti-UV, des agents antivieillissement, des agents antioxydants ; des agents de fluidisation, des agents anti-abrasion, des agents démoulants, des stabilisants ; des plastifiants, des modifiants chocs ; des agents tensioactifs ; des azurants ; des charges, telles que la silice, le noir de carbone, les nanotubes de carbone, le graphite expansé, l'oxyde de titane ou encore les billes de verre ; les fibres ; des cires ; et leurs mélanges. On pourra également envisager des additifs fonctionnels servant comme agent antimicrobien, bactéricide ou marqueurs laser.

La composition telle que définie plus haut peut avantageusement être utilisée pour constituer des granulés ou des poudres.

On utilise notamment lesdits granulés pour fabriquer un ballon de cathéter gonflable résistant à l'éclatement.

La présente invention a encore pour objet un procédé de fabrication d'un ballon de cathéter résistant à l'éclatement, ledit procédé comprenant :
- une étape de fourniture de copolymère conforme à celui défini dans l'une quelconque des revendications 1 à 9 ;
- une étape optionnelle de mélange dudit copolymère avec au moins un autre polymère et/ou au moins un additif, de façon à fabriquer une composition telle que définie à la revendication 10 ;
- une étape de mise en œuvre dudit copolymère ou de ladite composition à une température T0 comprise dans la gamme de 200 à 300°C ;
- une étape de formation du ballon à une température inférieure à la Tf (température de fusion) du copolymère ;
- une étape de récupération du ballon.

Avantageusement, le procédé selon l'invention comprend au moins l'une des étapes suivantes : « dry blend », extrusion, notamment co-extrusion, extrusion de tube, surmoulage (overmolding), soufflage, et leurs mélanges.

Selon un mode de réalisation particulier, le PEBA conforme à l'invention est utilisé pour la fabrication de la tige et d'un ballon de cathéter comprenant une ou plusieurs couches de composition telle que définie plus haut.

Dans un mode de réalisation préféré, le ballon est formé d'une seule couche polymère formé au moins en partie de PEBA « blocs longs » selon l'invention. Cependant, le ballon peut aussi comporter plusieurs couches dont au moins une couche est au moins en partie constituée de PEBA selon l'invention.

Le ballon préféré est formé de 100 % de PEBA selon l'invention. Cependant, le ballon peut être formé d'un mélange de PEBA avec un ou plusieurs matériaux polymères différents. Les matériaux polymères appropriés pour le mélange avec des PEBA « blocs longs » selon l'invention comprennent les polymères énumérés précédemment, couramment utilisés pour fabriquer des ballons pour cathéters de dilatation, tels que le polyamide ou du PEBA « blocs courts » non conforme à l'invention.

Le mélange de polymères préféré est un mélange de PEBA selon l'invention et de polyamide, dans lequel le pourcentage en poids préféré de polyamide est compris dans la gamme de 30% à 95% du poids total. Le polyamide préféré est le polyamide 11, le polyamide 12, ou leurs mélanges.

Dans le cas d'un ballon comportant plusieurs couches formées par co-extrusion, le PEBA selon l'invention peut être la couche interne ou la couche externe du ballon.

La présente invention a donc pour objet un élément gonflable, notamment un ballon ou ballonnet de cathéter résistant à l'éclatement, de composition conforme à l'invention, c'est-à-dire formé au moins en partie de la gamme particulière de copolymères à blocs polyéther et blocs polyamide (PEBA) sélectionnée selon l'invention.

Le ballon selon l'invention présente avantageusement une épaisseur de paroi comprise dans la gamme allant de 1 à 250 µm, de préférence de 2 à 60 µm, de préférence de 3 à 50 µm, de préférence de 4 à 40 µm.

La présente invention a également pour objet un cathéter comprenant un ballon conforme à celui pré-défini.

Différents modèles de cathéters à ballonnet bien connus dans le domaine peuvent être utilisés comme cathéter à ballon selon l'invention formé au moins en partie de PEBA. Le cathéter à ballonnet selon l'invention comprend par exemple un cathéter ayant un arbre allongé et un ballon gonflable formé au moins en partie de PEBA « blocs longs » selon l'invention sur une partie distale du cathéter.

Par exemple, le cathéter peut être un cathéter de dilatation classique pour angioplastie. En outre, le cathéter peut être utilisé pour poser un stent, d'abord monté sur le ballonnet du cathéter.

Le ballon selon l'invention à base de PEBA, combinant souplesse et résistance à la traction améliorées, permet de fournir des cathéters à ballon de profil fin, ayant une excellente aptitude à s'insérer dans les voies du système vasculaire du patient, à traverser la sténose, et à compresser la sténose pour ouvrir le vaisseau sanguin du patient.

Le ballon de l'invention peut être produit par des techniques classiques de fabrication d'éléments gonflables de cathéter, telles que le moulage par soufflage, et peut être préformé en étirant un tube droit avant que le ballon soit soufflé. Les ballons peuvent être formés par expansion de tube, par exemple dans un rapport de cercle compris entre 3 et 8. Le collage du ballonnet sur le cathéter peut se faire par des techniques classiques, telles que celles utilisant les adhésifs et/ou par fusion, avec éventuellement des compatibilisants. Le ballonnet peut être gonflé par un fluide radio-opaque à partir d'un orifice de gonflage situé dans la tige de cathéter, ou par d'autres moyens, notamment à partir d'un passage formé entre l'extérieur de la tige de cathéter et l'élément formant le ballon, en fonction de la conception particulière du cathéter. Les détails et les mécanismes de gonflage du ballonnet varient en fonction de la conception particulière du cathéter, et sont bien connus de l'homme du métier dans le domaine des cathéters. La longueur du ballonnet peut être d'environ 0,5 cm à environ 6 cm, de préférence d'environ 1,0 cm à environ 4,0 cm. Après avoir été formé, le ballonnet a par exemple un diamètre extérieur à la pression nominale (par exemple 6-8 ATM) d'environ 0,15 cm à environ 0,4 cm, et typiquement d'environ 0,3 cm, mais des ballons ayant un diamètre extérieur d'environ 1 cm peuvent également être utilisés. L'épaisseur de paroi simple est comprise dans la gamme de 1 à 250 µm, de préférence de 2 à 60 µm, de préférence de 3 à 50 µm, de préférence de 4 à 40 µm, par exemple d'environ 10 µm à environ 40 µm, et généralement de 15 µm. Dans le mode de réalisation dans lequel le ballonnet issu de coextrusion comporte deux couches, la couche de PA a de préférence une épaisseur de paroi unique de 5 à 15 µm, et la couche de PEBA a de préférence une épaisseur allant de 2 à 12 µm.

Selon un autre mode de réalisation de l'invention, un stent est disposé autour du ballon pour installation à l'intérieur d'un vaisseau d'un patient. Par exemple, le matériau de stent peut être en acier inoxydable, un alliage de NiTi, un matériau plastique ou d'autres matériaux. Le stent a un diamètre suffisamment petit pour permettre l'insertion et l'avancement dans les vaisseaux du patient, et peut se dilater à un diamètre plus large pour l'implantation dans les lumens du patient. Le ballon selon l'invention formé au moins en partie de PEBA présente, grâce au PEBA « blocs longs » selon l'invention, un meilleur glissant, utile pour la pose d'un stent. Dans le mode de réalisation de l'invention dans lequel le ballonnet a au moins deux couches co-extrudées, un ballonnet utilisé pour la pose de stent a de préférence la couche PEBA comme unique couche extérieure, pour fournir un meilleur glissant lors de la pose de l'endoprothèse vasculaire. En outre, la force de rétention du stent est améliorée lorsque le ballonnet est formé par co-extrusion.

### Exemples

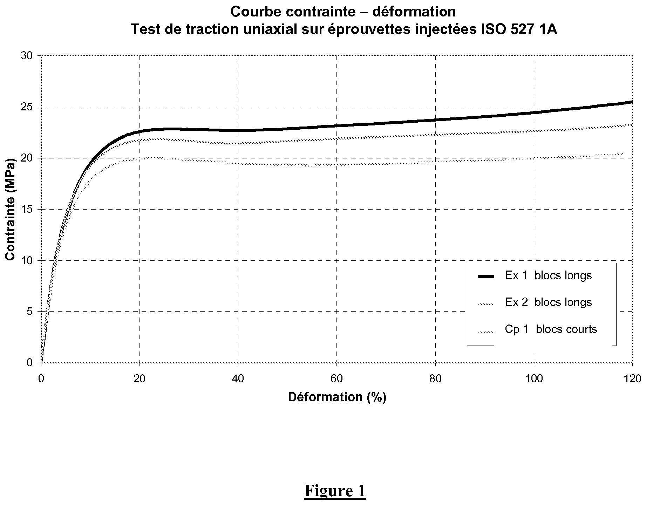

Les exemples ci-dessous illustrent la présente invention sans en limiter la portée. Dans les exemples, sauf indication contraire, tous les pourcentages et parties sont exprimés en poids.
Dans le tableau 1 suivant et le graphe de la figure 1, les propriétés de contrainte et de déformation sont mesurées lors d'un test de traction uniaxial sur éprouvettes injectées ISO 527 1A, conditions d'essai : 45°C, vitesse de déplacement du piston V= 50 mm / minute (selon la norme ISO 527, Zwick 3, extensomètre optique).

**Tableau 1**

| | **Taille des blocs PA12 - PTMG (g/mol) du PEBA** | **Viscosité inhérente** | **Dureté Shore D (ISO 868)** | **Déformation au seuil %** | **Contrainte au seuil (MPa)** | **Déformation au plateau d'écoulement( %)** | **Contrainte au plateau d'écoulement (MPa)** | **Contrainte à 100% déformation (MPa)** |
|---|---|---|---|---|---|---|---|---|
| **Ex 1** | 14000 - 650 | 1,53 | 74 | 25,8 | 22,8 | 40 | 22,7 | 24,4 |
| **Ex 2** | 14000 - 650 | 1,53 | 72 | 23,8 | 21,9 | 40 | 21,4 | 22,6 |
| **Cp 1** | 5000 - 250 | 1,38 | 72 | 21,8 | 20 | 55 | 19,3 | 20 |

Dans le tableau 2 suivant, le module élastique en flexion est mesuré à 23°C (échantillon conditionné). La norme utilisée ici est la norme internationale ISO 178, l'appareil de mesure consiste en un Zwick 1465, 2mm/min, capteur de déplacement.

**Tableau 2**

| | **Taille des blocs PA12 - PTMG du PEBA** | **Module d'élasticité en flexion à 23°C (MPa)** |
|---|---|---|
| **Ex 1** | 14000g/mol - 650g/mol | 625 |
| **Ex 2** | 14000g/mol - 650g/mol | 700 |
| **Cp 1** | 5000g/mol - 250g/mol | 525 |

Le graphe de la figure 1 et les tableaux 1 et 2 montrent que les ballons de cathéter utilisant les PEBA « blocs longs » conformes à l'invention (Ex 1 et Ex 2) présentent plusieurs propriétés mécaniques avantageuses par rapport aux structures similaires utilisant des PEBA « blocs courts » de l'art antérieur (Cp 1) :
- module élastique plus élevé ;
- meilleure résistance à la traction, donc pression d'éclatement plus élevée, mesurée par une contrainte et déformation au seuil plus élevées ;
- « compliance » améliorée marquée par :
- un « adoucissement moins marqué », c'est-à-dire des écarts inférieurs entre la contrainte et la déformation au seuil et la contrainte et la déformation au plateau d'écoulement ; ainsi que par
- un durcissement sous contrainte (ou rhéodurcissement) plus important, représenté par l'augmentation de la contrainte avec la déformation après le seuil de plasticité, c'est-à-dire en comparant la contrainte au seuil et la contrainte à 100% de déformation. Le seuil de plasticité a été mesuré selon la norme ISO 527:1 (point de donnée le plus élevé dans la région du seuil d'écoulement).

En définitive, on vérifie bien que le matériau PEBA selon l'invention est :
- résistant à l'éclatement, en particulier résistant à la traction, c'est-à-dire de contrainte au seuil supérieure à 20 MPa et de déformation au seuil supérieure à 22%, mesurée selon la norme ISO 527,
- et « compliant », c'est-à-dire dont l'écart entre la contrainte au seuil et la contrainte au plateau d'écoulement est inférieur à 0,5 MPa, et dont le rapport entre la déformation au seuil et la déformation au plateau d'écoulement est supérieur à 0,5, les contraintes et déformations étant mesurées selon la norme ISO 527 .

Le ballon selon l'invention, notamment celui formé à l'aide des PEBA de Ex 1 et Ex 2, présente une épaisseur de paroi uniforme et une résistance à la traction suffisante pour résister à la fois à la pression de gonflage nécessaire pour gonfler le ballonnet et pour compresser une sténose dans un vaisseau d'un patient.

## Revendications

1. Utilisation d'un copolymère à blocs polyéther et blocs polyamide pour la fabrication d'un élément gonflable de cathéter, tel qu'un ballon de cathéter, à résistance à l'éclatement améliorée, dans laquelle ledit copolymère a les caractéristiques suivantes :
- masse moléculaire moyenne en nombre des blocs PE supérieure à 500 g/mol,
- masse moléculaire moyenne en nombre des blocs PA supérieure à 10000 g/mol ; le copolymère comprend des blocs polyéther composés majoritairement à base de PTMG.

2. Utilisation selon la revendication 1, dans laquelle lesdits blocs PE représentent 1% à 10% en poids, sur le poids total du copolymère, et lesdits blocs PA représentent 80% à 99% en poids, de préférence de 90% à 99%, sur le poids total du copolymère.

3. Utilisation selon l'une quelconque des revendications précédentes, dans laquelle la dureté shore D (ISO 868) du copolymère est supérieure ou égale à 72, de préférence comprise dans la gamme de 72 à 76.

4. Utilisation selon l'une quelconque des revendications précédentes, dans laquelle le module d'élasticité en flexion (ISO 178) du copolymère est supérieur à 525 MPa, de préférence compris dans la gamme de 600 à 900 MPa.

5. Utilisation selon l'une quelconque des revendications précédentes, dans laquelle la masse moléculaire moyenne en nombre des blocs PE est supérieure à 600 g/mol, de préférence comprise dans la gamme de 600 à 2000 g/mol, de préférence de 650 à 1000 g/mol.

6. Utilisation selon l'une quelconque des revendications précédentes, dans laquelle la masse moléculaire en nombre des blocs PA est comprise dans la gamme de 12000 à 32000 g/mol, de préférence de 13000 à 25000 g/mol.

7. Utilisation selon l'une quelconque des revendications précédentes, dans laquelle les blocs PA sont formés à partir d'au moins un monomère choisi parmi : 6, 11, 12, 4.6, 4.12, 4.14, 4.18, 6.6, 6.10, 6.12, 6.14, 6.18, Pip.10, 9.6, 9.12, 10.10, 10.12, 10.14, 10.18, 10.36, 10.T, 6.T, 9.T, MXD.6, MXD.10, B.10, B.12, B.14, B.18, B.36, P.10, P.12, P.14, P.18, P.36, leurs copolymères statistiques et/ou à blocs, et leurs mélanges.

8. Utilisation selon l'une quelconque des revendications précédentes, dans laquelle ledit au moins un bloc PE comprend un polyéther choisi parmi les polyalkylènes éthers polyols, tels que le PEG, le PPG, le PO3G, les polyéthers contenant des séquences polyoxyalkylène à bouts de chaîne NH2, leurs copolymères statistiques et/ou à blocs, et leurs mélanges.

9. Utilisation selon les revendications 1 à 7, dans laquelle le copolymère comprend des blocs polyéther composés uniquement de PTMG.

10. Composition polymérique thermoplastique à base de polyamide, ladite composition comprenant :
- de 30 à 100% en poids de copolymère conforme à celui défini dans l'une quelconque des revendications 1 à 9,
- jusqu'à 70% en poids d'au moins un autre polymère choisi parmi les polyamides, TPU, PEBA autres que ceux définis dans l'une quelconque des revendications 1 à 9, COPE, PVC, ABS, PS, PET, PETE, PVDF, ETFE, Polyimide, PEEK, PEKK, silicone, « silicon rubber » (gomme de silicone), et
- jusqu'à 40% d'additif, sur le poids total de la composition.

11. Composition selon la revendication 10, dans laquelle l'additif est choisi parmi des traceurs (Ba2SO4, WC), des agents antimicrobien, bactéricides ou marqueurs laser, des agents colorants, notamment les pigments, les colorants, les pigments à effet, tels que les pigments diffractants, les pigments interférentiels, tels que les nacres, les pigments réfléchissants et leurs mélanges; des agents anti-UV, des agents antivieillissement, des agents antioxydant ; des agents de fluidisation, des agents anti-abrasion, des agents démoulant, des stabilisants ; des plastifiants, des modifiants chocs ; des agents tensioactifs ; des azurants ; des charges, telles que la silice, le noir de carbone, les nanotubes de carbone, le graphite expansé, l'oxyde de titane ou encore les billes de verre ; les fibres ; des cires ; et leurs mélanges.

12. Utilisation d'une composition telle que définie à l'une des revendications 10 ou 11 pour constituer des granulés ou des poudres.

13. Utilisation de granulés tels que définis à la revendication 12, pour la fabrication d'un ballon de cathéter gonflable à résistance à l'éclatement améliorée.

14. Procédé de fabrication d'un ballon de cathéter résistant à l'éclatement, ledit procédé comprenant :
- une étape de fourniture de copolymère conforme à celui défini dans l'une quelconque des revendications 1 à 9 ;
- une étape optionnelle de mélange dudit copolymère avec au moins un autre polymère et/ou au moins un additif, de façon à fabriquer une composition telle que définie à la revendication 10 ou 11 ;
- une étape de mise en œuvre dudit copolymère ou de ladite composition à une température T0 comprise dans la gamme de 200 à 300°C ;
- une étape de formation du ballon à une température inférieure à la Tf (température de fusion) du copolymère ;
- une étape de récupération du ballon.

15. Procédé selon la revendication 14, comprenant au moins l'une des étapes suivantes : « dry blend », extrusion, notamment co-extrusion, extrusion de tube, surmoulage (overmolding), soufflage, et leurs mélanges.

16. Utilisation d'un PEBA conforme à l'une quelconque des revendications 1 à 9, pour la fabrication d'un ballon de cathéter comprenant une ou plusieurs couches de composition conforme à l'une quelconque des revendications 10 ou 11.

17. Elément gonflable de cathéter, notamment ballon de cathéter, résistant à l'éclatement, **caractérisé en ce que** sa composition est conforme à l'une quelconque des revendications 10 ou 11.

18. Ballon selon la revendication 17 comprenant une épaisseur de paroi comprise dans la gamme allant de 1 à 250 µm, de préférence de 2 à 60 µm, de préférence de 3 à 50 µm, de préférence de 4 à 40 µm.

19. Cathéter comprenant un ballon conforme à l'une quelconque des revendications 17 ou 18.

## Patentansprüche

1. Verwendung eines Copolymers mit Polyetherblöcken und Polyamidblöcken zur Herstellung eines aufblasbaren Katheterelements, wie eines Katheterballons, mit verbesserter Berstfestigkeit, wobei das Copolymer die folgenden Eigenschaften aufweist:
- zahlenmittlere Molmasse der PE-Blöcke von mehr als 500 g/mol,
- zahlenmittlere Molmasse der PA-Blöcke von mehr als 10.000 g/mol;
wobei das Copolymer Polyetherblöcke umfasst, die hauptsächlich auf PTMG basieren.

2. Verwendung nach Anspruch 1, wobei die PE-Blöcke 1 bis 10 Gew.-%, bezogen auf das Gesamtgewicht des Copolymers, ausmachen und die PA-Blöcke 80 bis 99 Gew.- %, vorzugsweise 90 bis 99 %, bezogen auf das Gesamtgewicht des Copolymers, ausmachen.

3. Verwendung nach einem der vorhergehenden Ansprüche, wobei die Shore-D-Härte (ISO 868) des Copolymers größer oder gleich 72 ist und vorzugsweise im Bereich von 72 bis 76 liegt.

4. Verwendung nach einem der vorhergehenden Ansprüche, wobei der Biegeelastizitätsmodul (ISO 178) des Copolymers größer als 525 MPa ist und vorzugsweise im Bereich von 600 bis 900 MPa liegt.

5. Verwendung nach einem der vorhergehenden Ansprüche, wobei die zahlenmittlere Molmasse der PE-Blöcke größer als 600 g/mol ist und vorzugsweise im Bereich von 600 bis 2000 g/mol, vorzugsweise von 650 bis 1000 g/mol, liegt.

6. Verwendung nach einem der vorhergehenden Ansprüche, wobei die zahlenmittlere Molmasse der PA-Blöcke im Bereich von 12.000 bis 32.000 g/mol, vorzugsweise von 13.000 bis 25.000 g/mol, liegt.

7. Verwendung nach einem der vorhergehenden Ansprüche, wobei die PA-Blöcke aus mindestens einem Monomer ausgewählt aus 6, 11, 12, 4.6, 4.12, 4.14, 4.18, 6.6, 6.10, 6.12, 6.14, 6.18, Pip.10, 9.6, 9.12, 10.10, 10.12, 10.14, 10.18, 10.36, 10.T, 6.T, 9.T, MXD.6, MXD.10, B.10, B.12, B.14, B.18, B.36, P.10, P.12, P.14, P.18, P.36, statistischen Copolymeren und/oder Blockcopolymeren davon und Mischungen davon gebildet sind.

8. Verwendung nach einem der vorhergehenden Ansprüche, wobei der mindestens eine PE-Block einen Polyether umfasst, der aus Polyalkylenetherpolyolen, wie PEG, PPG, PO3G, Polyethern mit Polyoxyalkylensequenzen mit NH₂-Kettenenden, statistischen Copolymeren und/oder Blockcopolymeren davon und Mischungen davon ausgewählt ist.

9. Verwendung nach einem der Ansprüche 1 bis 7, wobei das Copolymer Polyetherblöcke umfasst, die ausschließlich aus PTMG bestehen.

10. Thermoplastische Polymerzusammensetzung auf Polyamidbasis, wobei die Zusammensetzung Folgendes umfasst:
- 30 bis 100 Gew.-% Copolymer gemäß der Definition in einem der Ansprüche 1 bis 9,
- bis zu 70 Gew.-% mindestens eines anderen Polymers, das aus Polyamiden, TPU, PEBA, die von denjenigen gemäß der Definition in einem der Ansprüche 1 bis 9 verschieden sind, COPE, PVC, ABS, PS, PET, PETE, PVDF, ETFE, Polyimid, PEEK, PEKK, Silikon, "Silicone Rubber" (Silikonkautschuk) ausgewählt ist, und
- bis zu 40 Gew.-% Additiv, bezogen auf das Gesamtgewicht der Zusammensetzung.

11. Zusammensetzung nach Anspruch 10, wobei das Additiv aus Tracern (Ba₂SO₄, WC), antimikrobiellen Mitteln, Bakteriziden oder Lasermarkern, Farbmitteln, insbesondere Pigmenten, Farbstoffen, Effektpigmenten, wie beugenden Pigmenten, Interferenzpigmenten, wie Perlglanzmitteln, reflektierenden Pigmenten und Mischungen davon, UV-Schutzmitteln, Alterungsschutzmitteln, Antioxidantien, Fließmitteln, Abriebschutzmitteln, Entformungsmitteln, Stabilisatoren, Weichmachern, Schlagzähigkeitsmodifikatoren, oberflächenaktiven Mitteln, optischen Aufhellern, Füllstoffen, wie Kieselsäure, Ruß, Kohlenstoffnanoröhren, Blähgraphit, Titanoxid oder auch Glasperlen, Fasern, Wachsen und Mischungen davon ausgewählt ist.

12. Verwendung einer Zusammensetzung gemäß einem der Ansprüche 10 oder 11 zum Aufbau von Granulaten oder Pulvern.

13. Verwendung von Granulaten gemäß Anspruch 12 zur Herstellung eines aufblasbaren Katheterballons mit verbesserter Berstfestigkeit.

14. Verfahren zur Herstellung eines berstfesten Katheterballons, wobei das Verfahren Folgendes umfasst:
- einen Schritt des Bereitstellens des Copolymers gemäß der Definition in einem der Ansprüche 1 bis 9;
- einen fakultativen Schritt des Mischens des Copolymers mit mindestens einem anderen Polymer und/oder mindestens einem Additiv zur Herstellung einer Zusammensetzung gemäß Anspruch 10 oder 11;
- einen Schritt des Verwendens des Copolymers bzw. der Zusammensetzung bei einer Temperatur T0 im Bereich von 200 bis 300 °C;
- einen Schritt des Bildens des Ballons bei einer Temperatur unterhalb der Tf (Schmelztemperatur) des Copolymers;
- einen Schritt der Gewinnung des Ballons.

15. Verfahren nach Anspruch 14, das mindestens einen der folgenden Schritte umfasst: "Dry Blend", Extrusion, insbesondere Coextrusion, Schlauchextrusion, Überspritzen (Overmolding), Blasformen und Mischungen davon.

16. Verwendung eines PEBA gemäß einem der Ansprüche 1 bis 9 zur Herstellung eines Katheterballons mit einer oder mehreren Schichten aus der Zusammensetzung gemäß einem der Ansprüche 10 oder 11.

17. Berstfestes aufblasbares Katheterelement, insbesondere berstfester Katheterballon, **dadurch gekennzeichnet, dass** seine Zusammensetzung einem der Ansprüche 10 oder 11 entspricht.

18. Ballon nach Anspruch 17 mit einer Wanddicke im Bereich von 1 bis 250 µm, vorzugsweise 2 bis 60 µm, vorzugsweise 3 bis 50 µm, vorzugsweise 4 bis 40 µm.

19. Katheter, umfassend einen Ballon gemäß einem der Ansprüche 17 oder 18.

## Claims

1. Use of a copolymer containing polyether blocks and polyamide blocks for manufacturing an inflatable catheter element, such as a catheter balloon, with improved bursting strength, in which said copolymer has the following characteristics:
- number-average molecular mass of the PE blocks greater than 500 g/mol,
- number-average molecular mass of the PA blocks greater than 10 000 g/mol;
the copolymer comprises polyether blocks composed predominantly on the basis of PTMG.

2. Use according to Claim 1, in which said PE blocks represent 1% to 10% by weight, relative to the total weight of the copolymer, and said PA blocks represent 80% to 99% by weight, preferably from 90% to 99%, relative to the total weight of the copolymer.

3. Use according to either of the preceding claims, in which the Shore D hardness (ISO 868) of the copolymer is greater than or equal to 72, preferably within the range from 72 to 76.

4. Use according to any one of the preceding claims, in which the flexural modulus of elasticity (ISO 178) of the copolymer is greater than 525 MPa, preferably in the range from 600 to 900 MPa.

5. Use according to any one of the preceding claims, in which the number-average molecular mass of the PE blocks is greater than 600 g/mol, preferably within the range from 600 to 2000 g/mol, preferably from 650 to 1000 g/mol.

6. Use according to any one of the preceding claims, in which the number-average molecular mass of the PA blocks is within the range from 12 000 to 32 000 g/mol, preferably from 13 000 to 25 000 g/mol.

7. Use according to any one of the preceding claims, in which the PA blocks are formed from at least one monomer chosen from: 6, 11, 12, 4.6, 4.12, 4.14, 4.18, 6.6, 6.10, 6.12, 6.14, 6.18, Pip.10, 9.6, 9.12, 10.10, 10.12, 10.14, 10.18, 10.36, 10.T, 6.T, 9.T, MXD.6, MXD.10, B.10, B.12, B.14, B.18, B.36, P.10, P.12, P.14, P.18, P.36, random and/or block copolymers thereof, and mixtures thereof.

8. Use according to any one of the preceding claims, in which said at least one PE block comprises a polyether chosen from polyalkylene ether polyols, such as PEG, PPG, PO3G, polyethers containing polyoxyalkylene sequences bearing NH2 chain ends, random and/or block copolymers thereof, and mixtures thereof.

9. Use according to Claims 1 to 7, in which the copolymer comprises polyether blocks solely composed of PTMG.

10. Polyamide-based thermoplastic polymer composition, said composition comprising:
- from 30% to 100% by weight of copolymer in accordance with that defined in any one of Claims 1 to 9,
- up to 70% by weight of at least one other polymer chosen from polyamides, TPU, PEBA other than those defined in any one of Claims 1 to 9, COPE, PVC, ABS, PS, PET, PETE, PVDF, ETFE, polyimide, PEEK, PEKK, silicone, "silicone rubber", and
- up to 40% of additive, relative to the total weight of the composition.

11. Composition according to Claim 10, in which the additive is chosen from tracers (Ba₂SO₄, WC), antimicrobial or bactericidal agents or laser markers, coloring agents, especially pigments, dyes, pigments with an effect, such as diffracting pigments, interference pigments, such as nacres, reflective pigments and mixtures thereof; UV stabilizers, antiaging agents, antioxidants; fluidizers, antiabrasion agents, mold-release agents, stabilizers; plasticizers, impact modifiers; surfactants; optical brighteners; fillers, such as silica, carbon black, carbon nanotubes, expanded graphite, titanium oxide or glass beads; fibers; waxes; and mixtures thereof.

12. Use of a composition as defined in either of Claims 10 and 11 for constituting granules or powders.

13. Use of granules as defined in Claim 12, for manufacturing an inflatable catheter balloon with improved bursting strength.

14. Process for manufacturing a burst-proof catheter balloon, said process comprising:
- a step of providing a copolymer in accordance with that defined in any one of Claims 1 to 9;
- an optional step of mixing said copolymer with at least one other polymer and/or at least one additive, so as to manufacture a composition as defined in Claim 10 or 11;
- a step of implementing said copolymer or said composition at a temperature T0 within the range from 200 to 300°C;
- a step of forming the balloon at a temperature below the Tm (melting point) of the copolymer;
- a step of recovering the balloon.

15. Process according to Claim 14, comprising at least one of the following steps: "dry blending", extrusion, especially coextrusion, tube extrusion, overmolding, blow-molding, and mixtures thereof.

16. Use of a PEBA in accordance with any one of Claims 1 to 9, for manufacturing a catheter balloon comprising one or more layers of composition in accordance with either of Claims 10 and 11.

17. Burst-proof inflatable catheter element, especially a catheter balloon, **characterized in that** its composition is in accordance with either of Claims 10 and 11.

18. Balloon according to Claim 17, comprising a wall thickness within the range of 1 to 250 µm, preferably from 2 to 60 µm, preferably from 3 to 50 µm, preferably from 4 to 40 µm.

19. Catheter comprising a balloon in accordance with either of Claims 17 and 18.
